(19) Europäisches Patentamt / European Patent Office / Office européen des brevets

(11) **EP 2 339 030 A1**

(12) **EUROPEAN PATENT APPLICATION**
published in accordance with Art. 153(4) EPC

(43) Date of publication:
**29.06.2011 Bulletin 2011/26**

(51) Int Cl.:
*C12Q 1/68* (2006.01)       *C12N 15/09* (2006.01)
*G01N 33/50* (2006.01)      *B01D 11/00* (2006.01)
*A61K 36/29* (2006.01)

(21) Application number: **09753423.4**

(22) Date of filing: **26.05.2009**

(86) International application number:
**PCT/CN2009/000587**

(87) International publication number:
**WO 2009/143705 (03.12.2009 Gazette 2009/49)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **30.05.2008  CN 200810109808**

(71) Applicant: **Golden Biotechnology Corporation Taiwan (CN)**

(72) Inventors:
• **LIU, Sheng-Yun**
 **Taipei County 25170 Taiwan (CN)**
• **WEN, Wu-Che**
 **Taipei County 25170 Taiwan (CN)**
• **KUO, Mao-Tien**
 **Taipei County 25170 Taiwan (CN)**

(74) Representative: **Cole, William Gwyn et al**
 **HLBBshaw**
 **Merlin House**
 **Falconry Court**
 **Baker's Lane**
 **Epping, Essex CM16 5DQ (GB)**

(54) **SCREENING METHOD FOR LIVER X RECEPTOR AGONISTS AND APPLICATION THEREOF**

(57)   A screening method for liver X receptor agonists.   By the method, Cimicifugae extracts are screened as LXR agonists.

FIG. 1

EP 2 339 030 A1

SEQUENCE LISTING

<110> Golden Biotechnology Corporation

<120> SCREENING METHOD FOR LIVER X RECEPTOR AGONIST AND APPLICATION THEREOF

<140> EP 09753423.4
<141> 2009-05-26

<150> CN200810109808.8
<151> 2008-05-30

<160> 6

<170> PatentIn Ver. 3.3

<210> 1
<211> 26
<212> DNA
<213> Artificial Sequence

<220>
<223> Exemplary forward primer based on a template in human liver cDNA library
<400> 1
aaggatccct gtcagaagaa cagatc                                        26


<210> 2
<211> 26
<212> DNA
<213> Artificial Sequence

<220>
<223> Exemplary reverse primer based on a template in human liver cDNA library

<400> 2
aaaagctttt cgtgcacatc ccagat                                        26


<210> 3
<211> 28
<212> DNA
<213> Artificial Sequence

<220>
<223> Exemplary forward primer based on a template of PhRL-TK


<400> 3
aagtcgacgg ccccgcccag cgtcttgt                                      28


<210> 4
<211> 44
<212> DNA
<213> Artificial Sequence

<220>
<223> Exemplary reverse primer based on a template of PhRL-TK

<400> 4
agatctgcgg cacgctgttg acgctgttaa gcgggtcgct gcag                    44


<210> 5

```
<211> 30
<212> DNA
<213> Artificial Sequence

<220>
<223> Exemplary forward primer based on a template of pG5SEAP


<400> 5
taccggttca cacaggaaac agctatgacc                                    30


<210> 6
<211> 26
<212> DNA
<213> Artificial Sequence

<220>
<223> Exemplary reverse primer based on a template of pG5SEAP


<400> 6
gggcgacacg gaaatgttga atactc                                        26
```

**Description**

FIELD OF THE INVENTION

The present invention relates to a screening method for identifying liver X receptor (LXR) agonists, and the LXR agonists screened by such method.

BACKGROUND OF THE INVENTION

Nuclear receptors (NR) form a superfamily capable of inducing transcription. Liver X receptors (LXRs) are members of the nuclear receptors superfamily, and liver and adipose tissues have large quantity of LXRs. In human bodies, LXRs are responsible for regulating and balancing metabolism of cholesterol, fatty acid, and glucose. Moreover, they are important regulators in the metabolism of lipids and lipoproteins. In mammals, two kinds of LXRs that are similar in structure but different in expression pattern are found. These two LXRs are liver X receptor alpha (LXR-$\alpha$) and liver X receptor beta (LXR-$\beta$) (Willy, P. J., Umesono, K., Ong, E. S., Evans, R. M., Heyman, R. A., and Mangelsdorf, D. J. 1995. LXR, a nuclear receptor that defines a distinct retinoid response pathway. Genes Dev. 9(9): 1033-45). LXR-$\alpha$ is mainly found in liver, and few metabolically active tissues or organs such as kidneys, small intestines, adipose tissues, and macrophages. On the other hand, LXR-$\beta$ is commonly found in tissues.

Typical NRs frequently function through the way described as follows. When specific ligands bind to NRs, the protein structure of the NRs changes. Then, the NRs are able to bind to the specific sequence of the promoter of the target gene, and thus, the expression of the gene occurs. Such signal transduction is called "activation". In the activation of LXRs to regulate lipometabolism, LXRs first bind to retinoic X receptors (RXRs) to form dimmers, and the dimmers enter the cell nucleus by the activation of cholesterol precursors and then bind to LXR response elements (LXRE) with specific recognition sequences on the promoter of the target gene. Consequently, the metabolism of lipid and cholesterol can be regulated to reduce the precipitation of the cholesterol in blood vessels. In addition, the product of the target gene activated by the LXRs can cause reverse cholesterol transport to form high-density lipoproteins (HDLs) from excessive cholesterol (Steffensen, K.R. and Gustafsson, J.A. 2004. Putative metabolic effects of the liver X receptor (LXR). Diabetes. 53 Suppl 1: S36-42). HDLs have various characteristics such as resistance to inflammation, oxidation, thrombosis, and induction of NO production, and can also efficiently decrease occurrence of cardiovascular diseases and atherosclerosis.

LXR agonists are a kind of substances that can bind to LXRs, initiate the function of LXRs, and increase the expression of the LXR target gene. For example, oxysterols, a kind of cholesterol precursors, are natural agonists that activate LXRs, and they include oxidative cholesterol derivatives such as 22(R)-hydroxycholesterol, 24(S)-hydroxycholesterol, 27-hydroxycholesterol, and cholestenoic acid. In addition, the artificially synthetic compound T0901317, chemically named as N-(2,2,2-trifluoroethyl)-N-[4-[2,2,2-trifluoro-1-hydroxy-1-(trifluoro-methyl)ethyl] phenyl]-benzenesulfonamide, is also a powerful LXR agonist.

According to the results of researches based on animal model experiments, it shows that the application of LXR agonists can be beneficial to the treatment of atherosclerosis and cardiovascular disease. Furthermore, it also shows these agonists are resistant to inflammation, and provide therapeutic effects on diabetes (Proctor, G., Jiang, T., Iwahashi, M., Wang, Z., Li, J. and Levi, M. 2006. Regulation of renal fatty acid and cholesterol metabolism, inflammation, and fibrosis in Akita and OVE26 mice with type 1 diabetes. Diabetes. 55(9): 2502-9), Alzheimer's disease (Koldamova, R. P., Lefterov, I. M., Staufenbiel, M., Wolfe, D., Huang, S., Glorioso, J. C., Walter, M., Roth, M. G. and Lazo, J. S. 2005. The liver X receptor ligand T0901317 decreases amyloid beta production in vitro and in a mouse model of Alzheimer's disease. J Biol Chem. 11,280(6): 4079-88), and even cancers (Fukuchi, J., Kokontis, J. M., Hiipakka, R.A., Chuu, C.P. and Liao, S. 2004. Antiproliferative effect of liver X receptor agonists on LNCaP human prostate cancer cells. Cancer Res. Nov 1, 64(21): 7686-9). Hence, it is desirable to provide a method for effectively screening agonists that can activate LXRs to benefit the treatment of atherosclerosis, cardiovascular diseases, and Alzheimer's disease, and, the prevention of diabetes and, moreover, the application to anti-inflammation.

SUMMARY OF THE INVENTION

In order to rapidly and efficiently find an agonist that activates LXRs, the present invention provides a screening method for identifying an LXR agonist, and by way of the invention method to evaluate whether Rhizoma Cimicifugae can efficiently activate the expression of LXRs or not, and also screen and identify agonists that activates LXRs for the prevention and treatment of atherosclerosis, cardiovascular diseases, and other diseases.

In one aspect of the invention methods, a fusion protein that is encoded by LXR-$\alpha$ ligand binding domain (LXR-$\alpha$ LBD) and GAL-4 DNA binding domain (GAL-4 BD), and a reporter gene such as secreted alkaline phosphatase (SEAP) that can be regulated by GAL-4 upstream activation sequence (GAL-4 UAS) are constructed together to form an $\alpha$-

SEAP expression plasmid. Then, the $\alpha$-SEAP expression plasmid is transfected into host cells such as Chinese hamster ovary cells (CHO-k1). In the process of the screening for LXR agonists, a test sample is added to the host cells culture medium and then measure the expression level of the reporter gene in the medium. The expression levels of the reporter gene before and after the addition of the test sample are compared. When the expression level of the reporter gene after the addition of the test sample is higher than that before addition, the test sample is identified as an LXR-$\alpha$ agonist.

In another aspect, the method of the present invention is further utilized to check whether Rhizoma Cimicifugae is a LXR agonist. Rhizoma Cimicifugae is a plant belonging to *Cimicifugeae* and the main medicinal part of the plant is rhizome. According to the description of traditional medical books, Rhizoma Cimicifugae exhibits effects such as clearing away heat and toxic materials, analgesia, anti-edema, sedation, and reducing symptoms of macula, and can be applied to treat some symptoms such as headache caused by a flu or cold, toothache, sore throat, and uterine prolapse. First, in accordance with the screening methods, the dry root skins or stems of the raw herbal materials of Rhizoma Cimicifugae and the commercially available scientific herbal medicine thereof are extracted with water and organic solvent respectively to prepare a water or organic solvent extract of the raw herbal materials and the scientific herbal medicine of Rhizoma Cimicifugae as test samples, which contain bioactive components. In some embodiments, the organic solvent used herein, without limitation, include alcohols (such as methanol, ethanol, or propanol), esters (such as ethyl acetate), alkanes (such as hexane), or haloalkanes (such as chloromethane and chloroethane. In some embodiments, the organic solvent is preferred to be an alcohol, and more preferred to be 70% ethanol.

Further in accordance with the invention screening methods, to the host cells culture medium, the above-prepared water and organic solvent extracts from the raw herbal materials and the scientific herbal medicine of Rhizoma Cimicifugae are added. When the test sample contains a ligand capable of binding to the LXR-$\alpha$ ligand binding domain, the expression of SEAP is driven to manifest. The analysis of the expression level of SEAP thus can evaluate if the raw herbal materials and the scientific herbal medicine of Rhizoma Cimicifugae are able to activate LXR efficiently. Consequently, it can be determined if the raw herbal materials and the scientific herbal medicine of Rhizoma Cimicifugae are LXR agonists.

Following the above mentioned screening platform of CHO-k1 in which the $\alpha$-SEAP expression plasmid is transfected, a variety of test samples can be screened rapidly and efficiently to determined the test sample that activates LXR-$\alpha$. Based on the experimental results, it was confirmed that the water and organic solvent extracts from the raw herbal materials and the scientific herbal medicine of Rhizoma Cimicifugae activate LXR-$\alpha$. Accordingly, the raw herbal materials and the scientific herbal medicine of Rhizoma Cimicifugae are LXR-$\alpha$ agonists and able to induce LXR-$\alpha$ to drive the expression of the downstream gene. Therefore, the metabolism of lipid and cholesterol can be regulated to achieve the prevention and treatment of atherosclerosis and cardiovascular diseases. Also, they can apply to the treatment of Alzheimer's disease, the prevention of diabetes, and the anti-inflammation.

Hereinafter, the embodiments of the present invention will be illustrated together with the drawings in more detail. The following examples are construed as the explanation for the present invention, but not as limitations for the present invention. One skilled in the art can make some changes and modifications without departing from the scope and spirit of the present invention. , The scope of the present invention may be in accordance with that of the accompanying claims.

BRIEF DESCRIPTION OF THE DRAWINGS

FIG. 1 shows an exemplary $\alpha$-SEAP expression plasmid in the example of the present invention.

FIG. 2 shows the illustrative activation fold of SEAP in the water and ethanol (70%) extracts from the scientific herbal medicine of Rhizoma Cimicifugae examined by the screening method for identifying LXR agonists in the example of the present invention.

FIG. 3 shows the illustrative activation fold of SEAP in the water and ethanol (70%) extracts from the raw herbal materials of Rhizoma Cimicifugae examined by the screening method for identifying LXR agonists in the example of the present invention.

FIG. 4 shows the illustrative $OD_{254}$ distribution of 180 fractions obtained from gel filtration chromatography where the water extract of the scientific herbal medicine of Rhizoma Cimicifugae was purified by a column of Sephadex LH-20 . The numbers from 1 to 14 means the groups classified from the 180 fractions according to the main peaks and the wavelength of the absorbance and the lines under the respective numbers mean the fraction ranges of the groups.

FIG. 5 shows the illustrative activation fold of SEAP in the 14 groups examined by the screening method for identifying LXR agonists in the example of the present invention. The 14 groups were classified from the 180 fractions obtained from gel filtration chromatography where the water extract of the scientific herbal medicine of Rhizoma Cimicifugae was purified by a column of Sephadex LH-20.

DETAILED DESCRIPTION OF THE PREFERRED EMBODIMENT

A cell line transfected with an α-SEAP expression plasmid used for screening and identifying LXR agonists is prepared. During the culture of the cell line, the mock control, the negative control, the positive control (T0901317), and the test samples are added in the media, respectively. If the test samples are LXR agonists, the expression of the reporter gene on the α-SEAP expression plasmid is driven. The samples tested in the example of the present invention are the water and organic solvent extracts from the raw herbal materials and the scientific herbal medicine of Rhizoma Cimicifugae. The raw herbal materials and the scientific herbal medicine of Rhizoma Cimicifugae are extracted respectively with water and organic solvent according to a conventional method for extraction to give a water or organic solvent extract of the raw herbal materials and the scientific herbal medicine of Rhizoma Cimicifugae. The organic solvents used in the extraction in some instances, include but are not limited to, alcohols (such as methanol, ethanol, or propanol), esters (such as ethyl acetate), alkanes (such as hexane), or haloalkanes (such as chloromethane and chloroethane). In some embodiments, the organic solvent is preferably an alcohol, and more preferably ethanol. In addition, based on the increased expression level of the reporter gene in the following examples of the present invention, LXRs can be efficiently activated by both the water and organic solvent extracts from the raw herbal materials and the scientific herbal medicine of Rhizoma Cimicifugae. Compared with the activated level of LXRs by the positive control (T0901317), it can be known that the screening platform of the α-SEAP expression plasmid constructed in the present invention can efficiently and accurately screen and select agonists which are able to activate LXRs effectively. This platform is beneficial to rapidly screen and identify agonists that are able to activate LXRs. The abovementioned methods are described in detail as follows:

**Example 1 Construction of α-SEAP expression plasmid**

In order to rapidly screen and identify an agonist that activates LXRs, an α-SEAP expression plasmid was constructed for screening and identifying an LXR agonist. The expression plasmid included a fusion protein that is encoded by LXR-α ligand binding domain (LXR-α LBD) and GAL-4 DNA binding domain (GAL-4 BD), and secreted alkaline phosphatase (SEAP) that can be regulated by GAL-4 upstream activation sequence (GAL-4 UAS).

SEAP was obtained from the modification of human placental alkaline phosphatase (PLAP). The best advantage of the SEAP reporter gene is ability to be secreted outside the cells (e.g., into the cell culture medium), and thus a portion of the medium can be used for a nondestructive enzyme activity assay. After the assay, the medium can be used for other researches.

GAL4 is a transcriptional activator of yeast and can start the gene of galactosidase (gall) to be transcribed so as to activate galactose metabolism. GAL4 consists of GAL-4 BD at the N-terminus and GAL-4 activation domain (GAL-4 AD) at the C-terminus, and these two domains have relatively independent functions and are structurally separated. GAL-4 BD can recognize GAL-4 UAS located on the GAL4 effector and binds thereto. GAL-4 AD can bind to other components of the transcription complex, and activates the transcription of the downstream gene of GAL-4 UAS.

When the α-SEAP expression plasmid was constructed, the template is based on the human liver cDNA library. The exemplary primers LXR-α-LBD-2-F (SEQ ID NO: 1) and LYR-α-LBD-1-R (SEQ ID NO: 2) were used for polymerase chain reaction (PCR). The sequences of the exemplary primers are listed as follows:

(1) Forward primer: LXR-α-LBD-2-F (SEQ ID NO: 1)
5'-AAGGATCCCTGTCAGAAGAACAGATC-3'
(2) Reverse primer: LXR-α-LBD-1-R (SEQ ID NO: 2)
5'-AAAAGCTTTTCG TGCACATCCCAG AT-3'

After the product of PCR was purified, the product was treated with the restriction enzymes *BamH*I and *Hind*III and ligated to pCMV-BD (purchased from Stratagene) previously treated with the restriction enzymes *BamH*I and *Hind*III. Subsequently, phRL-TK (purchased from Promega) was used as the template and mTK-1-F (SEQ ID NO: 3) and mTK-2-R (SEQ ID NO: 4) were used to serve as the exemplary primers in PCR. The sequences of the exemplary primers are listed as follows:

(3) Forward primer: mTK-1-F (SEQ ID NO: 3)
5'-AAGTCG ACGGCCCCGCCCAGCGTCTTGT-3'
(4) Reverse primer: mTK-2-R (SEQ ID NO: 4)
5'-AGATCTGCGGCACGCTGTTGACGCTGTTAAGCGGGTCGCTGCAG-3'

After the product of PCR was purified, the product was ligated to pGEM easy vector (purchased from Promega), digested with the restriction enzymes *Sph*I and *Sal*I, and then ligated to pG5SEAP vector (purchased from BD Biosciences). Abovementioned pGSSEAP (containing TK) was used to serves as the template, and SEAP cassette F and Beta-lactamase R were used as the primers in PCR. The sequences of the exemplary primers are listed as follows:

(5) Forward primer: SEAP cassette F (SEQ ID NO: 5)
5'-TACCGGTTCACACAGGAAACAGCTATGACC-3'
(6) Reverse primer: β-lactamase R (SEQ ID NO: 6)
5'-GGGCGACACGGAAATGTTGAATACTC-3'

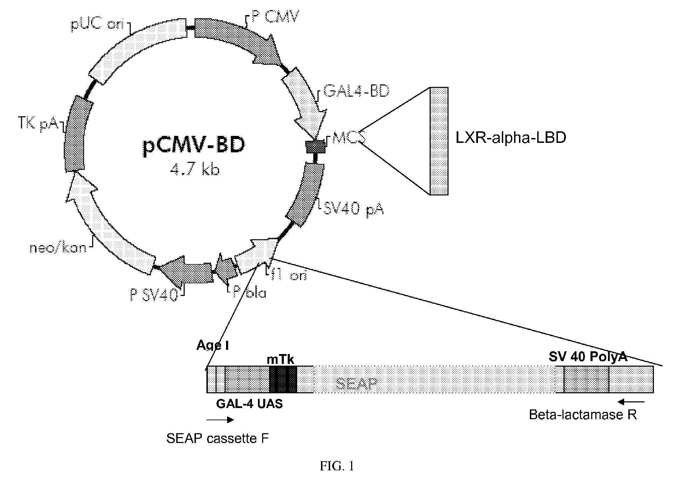

After the product of PCR was purified, the product was digested with the restriction enzyme DraIII and ligated to pCMV-BD LXR-α LBD previously treated with the restriction enzyme DraIII. The product of PCR was inserted at the replication start point (f1 ori) of the plasmid to prepare the α-SEAP expression plasmid as shown in FIG. 1.

**Example 2 Construction of screening method for LXR agonists**

The α-SEAP expression plasmid prepared in Example 1 was used in the screening method to identify LXR agonists. The method includes the following steps:

(a) constructing an expression plasmid which comprises at least a sequence of LXR-α ligand binding domain, a sequence of GAL-4 DNA binding domain, a GAL-4 upstream activation sequence recognized by the GAL-4 DNA binding domain, and a reporter gene regulated by the GAL-4 upstream activation sequence;
(b) transfecting the expression plasmid to host cells in the cell culture medium;
(c) adding a test sample to a host cells culture medium;
(d) measuring an expression level of the reporter gene in the culture medium;
(e) comparing the expression levels of the reporter gene before and after the addition of test sample; and
(f) identifying the test sample as an LXR-α agonist when the expression level of the reporter gene after addition of the test sample is higher than that therebefore in the step (e), which indicates the test sample comprises a ligand capable of binding to the LXR-α ligand binding domain.

In some embodiments, the expression plasmid of the step (a) is the α-SEAP expression plasmid described herein or other expression plasmids comprising the sequence elements listed in the step (a). In some embodiments, the reporter gene is SEAP described herein or other reporter genes that are easily detectable and have sensitive, quantifiable, and/or repeatedly assayed characteristics such as β-galatosidase or fluorescent proteins (e.g., luciferase), but is not limited thereto. In some embodiments, in the step (b), the host cells can express the fusion protein encoded by GAL-4 BD and LXR-α LBD. In some embodiments, in the step (c), when the test sample comprises a ligand capable of binding to LXR-α LBD, GAL-4 BD binds to GAL-4 UAS and then the expression of the reporter gene on the α-SEAP expression is driven. The expression of the reporter gene can be used to evaluate whether the test samples activate LXRs. Accordingly, LXR agonists can be obtained by the screening method described in the present invention.

**Example 3 Tests used in the screening method of LXR agonists**

The present experiment employed the potent artificial agonist T0901317 to evaluate the applicability of the exemplary screening method for identifying LXR agonists of Example 2 and to determine if Rhizoma Cimicifugae is an LXR agonist. The α-SEAP expression plasmid constructed above for the screening of LXR agonists was transfected into a cell line. During the culture of the cell line, the positive control T0901317 and the extracts of the raw herbal materials and the scientific herbal medicine of Rhizoma Cimicifugae prepared below were added to the cell culture media. When the test samples comprise a ligand capable of binding to LXR-α LBD, the expression of the reporter gene on the α-SEAP expression plasmid is driven. In addition, the expression level of the reporter gene was measured to identify whether the test samples are LXR agonists. The details of the experiment are depicted as follows.

**(1) Extraction of active components from Rhizoma Cimicifugae**

In the present experiment, the raw herbal materials and the scientific herbal medicine of Rhizoma Cimicifugae were used for extraction. In the raw herbal materials of Rhizoma Cimicifugae, dry root skins or stems were crushed and ground. The raw herbal materials included *Cimicifuga heracleflolia* Kom., *Cimicifuga dahurica* (Turcz.) Maxim., or *Cimicifuga foetida* L. Among these species, *C. heracleflolia* Kom. exists in Northeast China; *C. dahurica* (Turcz.) Maxim. exists in Northeast China, Northern China, and Central China; and *C. foetida* L. exists in Southern China, Central China, Northern China, and Western China. The commercially available scientific herbal medicine of Rhizoma Cimicifugae (purchased from Sun Ten Pharmaceutical Co., Ltd.) consists of the single extract of *C. foetida* L., and 1 gram thereof contains 0.68 g extract in which partial water is removed after the decoction of *C. foetida* L. and 0.32 g excipient. The raw herbal materials and the scientific herbal medicine of Rhizoma Cimicifugae were extracted respectively with water and organic solvent. In the water extraction, double deionized water (ddH$_2$O) was added in an amount of 10 times the volume of the herbal materials. The mixture was mixed by rotation for 4 hours and then kept overnight in a refrigerator (4°C). The supernatant was collected after centrifugation of the mixture. After the extract obtained thereby was lyophilized, the recovery ratio was calculated. In the organic solvent extraction, 70% ethanol is added in an amount of 10 times the volume of the herbal materials. The mixture was mixed by rotation for 4 hours and then kept overnight in a refrigerator (4°C). Thereafter, the supernatant was collected after centrifugation of the mixture. After the extract obtained thereby was lyophilized, the recovery ratio was calculated. The water and ethanol extracts from the extraction of the raw herbal materials and the scientific herbal medicine of Rhizoma Cimicifugae were stored to serve as test samples for the subsequent screening.

**(2) Determining whether the extracts of Rhizoma Cimicifugae are LXR agonist by the screening method of LXR agonist**

The α-SEAP expression plasmid prepared in Example 1 was transfected into the Chinese hamster ovary cell line (CHO-k1). The cell line successfully transfected was cultured in a suitable medium for 5 hours in a CO$_2$ incubator at 37°C. Subsequently, the water and ethanol extracts from the raw herbal materials and the scientific herbal medicine of Rhizoma Cimicifugae at the concentrations of 0.1 μg/ml, 1 μg/ml, and 10 μg/ml were added respectively. In addition, the cell culture medium to which the α-SEAP expression plasmid was not transfected with no test samples added was used as a mock control; the cell culture medium to which no test samples added was used as a negative control; and the cell culture medium containing 2 μM T0901317 (purchased from Merck, USA) was used as a positive control. T0901317, chemically named as N-(2,2,2- trifluoroethyl)-N-[4-[2,2,2-trifluoro-1-hydroxy-1-(trifluoromethyl)ethyl] phenyl]-benzenesulfonamide has been reported as an LXR-α and LXR-β agonist and can induce the expression of *abc1* gene (mediating cholesterol transport) by activation of the heterodimerization between LXR and RXR receptors to promote cholesterol efflux from intestinal cells. As a result, the absorption of cholesterol can be inhibited in the intestines so as to achieve resistance to atherosclerosis. Accordingly, T0901317 was used to serve as a positive control in the experiment to determine the performance of the screening method constructed in the present invention.

After the test samples were added into the culture media for 24 to 48 hours, the media were collected for detection of the cell viability and the activity of the reporter gene SEAP. The cell viability was determined by the MTT assay. The MTT method is a common method for biologically determining the cell viability or proliferation. In the MTT method, succinate dehydrogenase in the mitochondria of live cells can transform tetrazolium of MTT into blue formazan accumulated in the cells. DMSO added can dissolve formazan and then the OD value is measured to determine the performance of the MTT reduction in the cells (i.e. the formation of formazan). This OD value indicates the activity of the mitochondria, i.e. the number of the cells. In this assay, MTT (2.5 mg/ml) was added to the treated cells in the dark for 4 hours, and then lysis buffer (100 μl) was added in each well of the cells. Finally, the absorbance (570 nm) was measured by an ELISA reader for subsequent calculation of the cell viability.

The activity of the reporter gene SEAP was determined by the absorbance. In the method, each cell culture medium was collected in an eppendorf (1.5 ml), heated at 65°C for 5 minutes, and centrifuged at 10,000 rpm for 15 minutes. Then, each test sample was added to each well of a 96-well microplate for the subsequent activity test. Basically, different test samples may have different activities. In order to prevent the absorbance of the over-sampling volume from going beyond a linear range owing to a relatively high activity of the test samples or to prevent the absorbance of the under-sampling volume from becoming undetectable owing to a relatively low activity of the test samples, each of the test samples was assayed with low, medium, and high amounts, and such sampling could also achieve testing in triplicate. The sampling volumes of each test sample were 10 μl, 20 μl, and 50 μl. Then, double deionized water (ddH$_2$O) was added until the total volume reached 100 μl. Finally, the coloring reagent (100 μl) in which pNPP (40 mg) was dissolved in the 2X SEAP buffer (8 ml) was added. The absorbance A$_{405}$ was measured every 5 minutes for 60 minutes according to the kinetic mode, and the variation of the SEAP activity was calculated according to the following equation.

$$\text{SEAP activity (mU/ml)} = [(A_{450} \text{ at 60 minute} - A_{450} \text{ at 0 minute})/60/0.04] \times$$

$$(1000/\text{sampling volume})$$

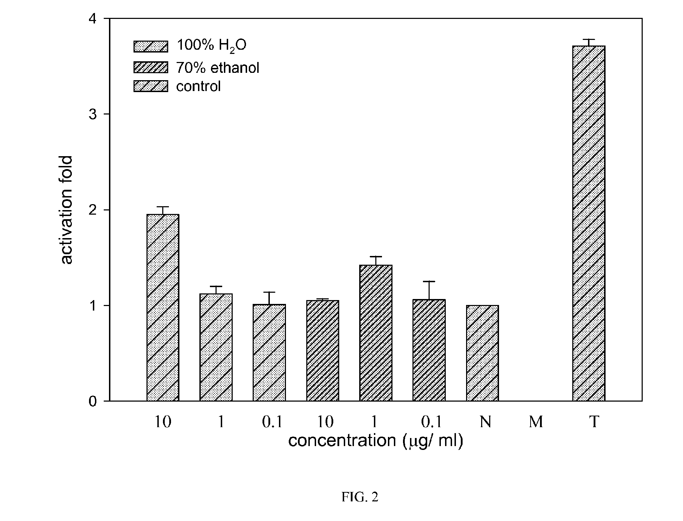

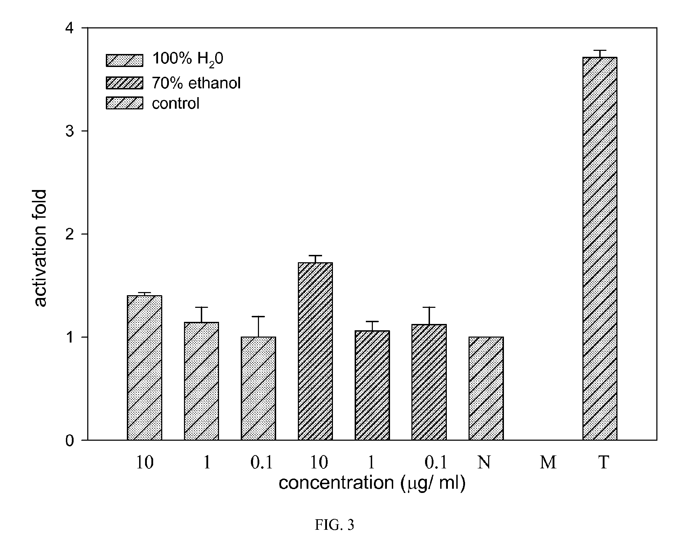

In the equation, 1 mU (milliunit) represents the increase of the absorbance A$_{405}$ per 0.04 minute. In addition, in order to confirm that the SEAP activity is incurred from the effect of the medicine but not from the variation of the cell number, the activities measured were standardized by the cell viability in the present invention. That is, the measured SEAP activity is divided by the OD value of MTT, and the obtained activity are the activity per unit cell. Such procedures are the standardization of the SEAP activity. Furthermore, the ratios of the values of the test samples to the negative control were calculated and these ratios represent the activation fold of LXRs. The results are shown in FIGs. 2 and 3.

Referring to FIGs. 2 and 3, FIG. 2 shows the activation fold of SEAP in the water and ethanol (70%) extracts respectively from the scientific herbal medicine of Rhizoma Cimicifugae examined by the invention screening method for identifying LXR agonists. FIG. 3 shows the activation fold of SEAP in the water and ethanol (70%) extracts respectively from the raw herbal materials of Rhizoma Cimicifugae examined by the screening method for identifying LXR agonists. As shown in FIG. 2, the activation folds of SEAP in various concentrations of the water and ethanol extracts from the scientific herbal medicine of Rhizoma Cimicifugae are all higher than that of the negative control (N). This indicates all of these extracts can activate LXRs and increase the LXR expression. Among these extracts, the water extract of the scientific herbal medicine of Rhizoma Cimicifugae at the concentration of 10 $\mu$g/ml exhibits the highest activation fold, and this activation fold is twice as high as that of the negative control. As shown in FIG. 3, except for the activation fold of the 0.1 $\mu$g/ml water extract of the raw herbal materials of Rhizoma Cimicifugae which is similar to that of the negative control, the water and ethanol extracts of the raw herbal materials of Rhizoma Cimicifugae at the other concentrations exhibit the higher activation fold than that of the negative control. This indicates the water extract of the raw herbal materials of Rhizoma Cimicifugae at the concentration higher than 0.1 $\mu$g/ml can efficiently activate the $\mu$LXR expression and the ethanol extract thereof at the concentration from 0.1 $\mu$g/ml to 10 $\mu$g/ml all can activate LXRs and increase the LXR expression. Among these extracts, the ethanol extract of the raw herbal materials of Rhizoma Cimicifugae at the concentration of 10 $\mu$g/ml exhibits the highest activation fold, and this activation fold is 1.7 times as high as that of the negative control. These results demonstrates the water and ethanol extracts from the raw herbal materials and the scientific herbal medicine of Rhizoma Cimicifugae may contain ligand(s) capable of binding to the LXR-$\alpha$ LBD and can be used as an agonist that activates LXRs.

On the other hand, as shown in FIGs. 2 and 3, it can be found that the positive control of the potent LXR agonist T0901317 exhibits the significantly higher activation fold than that of the negative control. This expected result of the activation fold demonstrates the invention screening method for identifying LXR agonists utilizing the CHO-k1 cells transfected by $\alpha$-SEAP expression plasmid can efficiently screen and select the agonist that activates LXRs. Furthermore, in accordance with the invention screening method, the test samples are identified as LXR agonists as long as the activation fold of the reporter gene in the test samples is higher than that of the negative control and/or a positive correlation is present between the activity and the concentration of the test samples.

Moreover, when compared the activation folds of the water and ethanol extracts from the raw herbal materials with those of the scientific herbal medicine of Rhizoma Cimicifugae, it was found that the water extract of the scientific herbal medicine of Rhizoma Cimicifugae at the concentration of 10 $\mu$g/ml exhibits the significantly higher SEAP activation fold than that of the other extracts. Therefore, in the present invention, the water extract of the scientific herbal medicine of Rhizoma Cimicifugae at the concentration of 10 $\mu$g/ml was further purified with gel filtration chromatography. After that, each fraction was further screened according to the invention screening method to find out which components activate LXRs.

### (3) Utilizing the screening method for identifying LXR agonists to find out active components that activate LXRs in the water extract of the scientific herbal medicine of Rhizoma Cimicifugae

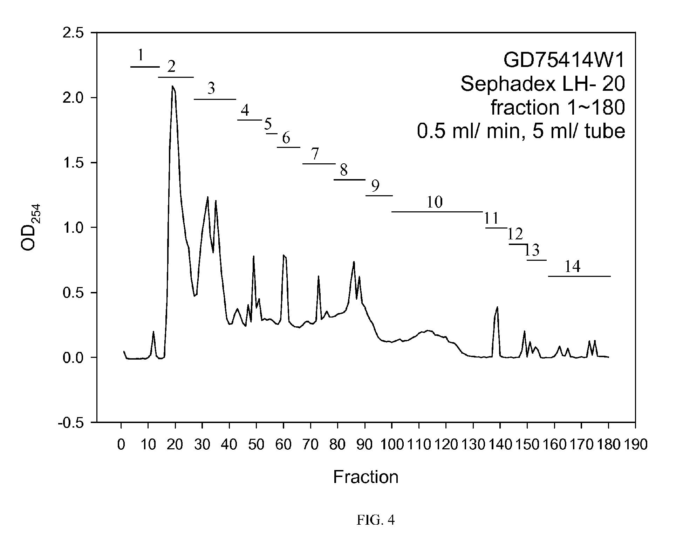

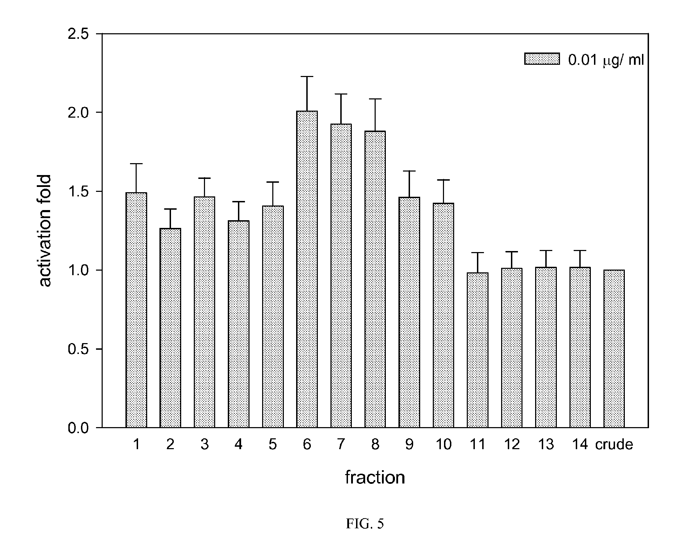

10 g of scientific herbal medicine of Rhizoma Cimicifugae was dissolved in water (100 ml) for extraction. After extraction and centrifugation, the supernatant was lyophilized to give 2.4 g (n/w), then dissolved in water (8 ml), and purified by a column of Sephadex LH-20 for gel filtration chromatography. Sephadex LH-20 achieves separation according to the particle size of the gel packed in the column and the molecular weight of the extracts. In the column of Sephadex LH-20, deionized water was first used in an amount of three times as much as the volume of the gel for equilibrium. The water extract (8 ml, 300 $\mu$g/ml) of the scientific herbal medicine of Rhizoma Cimicifugae was added into the column. The purification is performed with double deionized water as the eluent at the flow rate of 0.5 ml/min. The eluate was collected into 180 fractions of which each was 5 ml. To a quartz plate, 200 $\mu$l of each fraction collected above was added and $OD_{254}$ thereof was measured with an ELISA reader. The $OD_{2s4}$ distribution of each fraction is shown in FIG. 4. Based on the $OD_{254}$ distribution shown in FIG. 4, a certain region that is significantly distinguished from the others, is construed as a main peak. According to the number of the main peaks, the 180 fractions were classified into 14 groups. The distribution range of the main peaks and $OD_{254}$ of the 14 groups are listed in Table 1. The 14 groups were screened at the concentration of 0.01 $\mu$g/ml by the invention screening method for identifying LXR agonists to determine whether there was any group containing active components that activate LXRs. The activity of SEAP in each group was measured. Then, the ratio of the SEAP activity of each group to that of the water extract (0.01 $\mu$g/ml) of the scientific herbal medicine of Rhizoma Cimicifugae was calculated. This ratio is the activation fold to LXRs, and the result thereof is shown in FIG. 5.

Table 1 Peak distribution range and $OD_{254}$ of 14 groups

| Group | Peak distribution range | $OD_{254}$ range |
|---|---|---|
| 1 | 1st to 15 th in the 180 fractions | 0.0457~ -0.00955 |
| 2 | 16th to 2th in the 180 fractions | 0.00125~ 0.4714 |
| 3 | 28th to 40th in the 180 fractions | 0.4850~ 0.2529 |
| 4 | 41st to 51st in the 180 fractions | 0.2599~ 0.4509 |
| 5 | 52nd to 57th in the 180 fractions | 0.2843~ 0.2612 |
| 6 | 58th to 66th in the 180 fractions | 0.2532~ 0.2294 |
| 7 | 6th to 77th in the 180 fractions | 0.2467~ 0.311 |
| 8 | 78th to 87th in the 180 fractions | 0.3112~ 0.4479 |
| 9 | 88th to 100th in the 180 fractions | 0.6201~ 0.1151 |
| 10 | 101st to 137th in the 180 fractions | 0.1226~ 0.0039 |
| 11 | 138th to 141st in the 180 fractions | 0.3121~ 0.002 |
| 12 | 142nd to 147th in the 180 fractions | -0.0005~ 0.0003 |
| 13 | 148th to 156th in the 180 fractions | 0.0472~ -0.0054 |
| 14 | 157th to 180th in the 180 fractions | -0.0021~ 0.0013 |

With reference to FIG. 5, it shows the activation fold of SEAP in the 14 groups examined by the invention screening method for LXR agonists, wherein the 14 groups were purified from the water extract of the scientific herbal medicine of Rhizoma Cimicifugae through Sephadex LH-20. Based on FIG. 5, in the respective groups purified from the water extract of the scientific herbal medicine of Rhizoma Cimicifugae through Sephadex LH-20, except that the SEAP activation fold of the 11th to 14th groups is close to that of the crude water extract of the scientific herbal medicine of Rhizoma Cimicifugae, the SEAP activation fold of the other groups (1st to 10th groups) is relatively higher than that of the crude water extract. Among these ten groups, the SEAP activation fold of the 6th group is the highest and about twice as high as that of the water extract of the scientific herbal medicine of Rhizoma Cimicifugae. This result indicates the 6th group (i.e. the collected 58th to 66th factions in the 180 fractions) purified from the water extract of the scientific herbal medicine of Rhizoma Cimicifugae through Sephadex LH-20 has better activation to LXRs.

Hence, the water and ethanol (70%) extracts of the raw herbal materials and the scientific herbal medicine of Rhizoma Cimicifugae and the 6th group of the fractions mentioned above which all have the ability of activating LXRs, can induce the expression of the LXR downstream gene to prevent atherosclerosis and cardiovascular diseases. In addition, these substances comprising components that efficiently activate LXR, in some embodiments, can also be used as agonists activating LXRs and manufactured into a pharmaceutical composition for medical applications for anti-atherosclerosis and anti-inflammation, and prevention of diabetes. In addition to the substances in an effective amount, the invention pharmaceutical compositions can further comprise a pharmaceutically acceptable carrier. The carrier, without limitation, can be a vehicle (e.g., water), a filler (e.g., sucrose or starch), a binder (e.g., cellulose derivatives), a diluent, a disintegrating agent, an absorption enhancer, or a sweetener. The invention pharmaceutical compositions, in some embodiments, are prepared according to common practices known in the medical art. In some embodiments, the substances described herein in an effective amount are mixed with one or more carriers to give a desired drug form. The drug form may include a tablet, powder, a granule, a capsule, or other liquid forms, but not be limited thereto.

**Claims**

1. A screening method for identifying a liver X receptor alpha (LXR-α) agonist comprising the following steps:

   (a) constructing an expression plasmid which comprises at least a sequence of LXR-α ligand binding domain, a sequence of GAL-4 DNA binding domain, a GAL-4 upstream activation sequence recognized by the GAL-4 DNA binding domain, and a reporter gene regulated by the GAL-4 upstream activation sequence;
   (b) transfecting the expression plasmid to host cells in a cell culture medium;
   (c) adding a test sample to the host cells culture medium;
   (d) measuring an expression level of the reporter gene in the cell culture medium;
   (e) comparing the expression levels of the reporter gene before and after addition of the test sample; and
   (f) identifying the test sample as an LXR-α agonist when the expression level of the reporter gene after addition of the test sample is higher than that therebefore in the step (e), which indicates that the test sample comprises a ligand capable of binding to the LXR-α ligand binding domain.

2. The method as claimed in claim 1, wherein a product of the reporter gene is an enzyme or a fluorescent protein that is detectable and quantifiable.

3. The method as claimed in claim 1, wherein the reporter gene is a secreted alkaline phosphatase (SEAP).

4. The method as claimed in claim 3, wherein the expression level of the reporter gene is determined by analysis of an ELISA reader.

5. The method as claimed in claim 1, wherein the host cells are mammalian cells.

6. The method as claimed in claim 5, wherein the mammalian cells are Chinese hamster ovary cells (CHO-k1).

7. A use of a Cimicifugeae extract to manufacture a medicament for activating liver X receptor alpha (LXR-α), wherein the Cimicifugeae extract is isolated from a raw herbal material or scientific herbal medicine of Rhizoma Cimicifugae.

8. The use as claimed in claim 7, wherein the raw herbal material of Rhizoma Cimicifugae is selected from a group consisting of *Cimicifuga heracleflolia* Kom., *cimicifuga dahurica* (Turcz.) Maxim., and *cimicifuga foetida* L.

9. The use as claimed in claim 7, wherein the scientific herbal medicine of Rhizoma Cimicifugae consists of the single extract of *cimicifuga foetida* L.

10. The use as claimed in claim 8 or 9, wherein the Cimicifugeae extract is an alcohol extract.

11. The use as claimed in claim 10, wherein the alcohol is ethanol.

12. The use as claimed in claim 11, wherein a concentration of the ethanol is 70%.

13. The use as claimed in claim 8 or 9, wherein the Cimicifugeae extract is a water extract.

14. An agonist for activating liver X receptor alpha (LXR-$\alpha$) comprising at least an effective amount of ethanol extract of a raw herbal material of Rhizoma Cimicifugae and a pharmaceutically acceptable carrier, wherein the raw herbal material of Rhizoma Cimicifugae is selected from a group consisting of *cimicifuga heracleflolia* Kom., *cimicifuga dahurica* (Turcz.) Maxim., and *cimicifuga foetida* L.

15. An agonist for activating liver X receptor alpha (LXR-$\alpha$) comprising at least an effective amount of water extract of a scientific herbal medicine of Rhizoma Cimicifugae and a pharmaceutically acceptable carrier, wherein the scientific herbal medicine of Rhizoma Cimicifugae consists of the single extract of *cimicifuga foetida* L.

16. A method of manufacturing a Cimicifugeae extract for activating liver X receptor alpha (LXR-$\alpha$), comprising the following steps:

    (a) taking 8 ml of a 300 $\mu$g/ml water extract of a scientific herbal medicine of Rhizoma Cimicifugae into a column of Sephadex LH-20 for gel filtration chromatography at a flow rate of 0.5 ml/min, and collecting 180 fractions of eluate in 5 ml each, wherein the scientific herbal medicine of Rhizoma Cimicifugae consists of the single extract of *cimicifuga foetida* L.;
    (b) measuring absorbance of the fractions with an ELISA reader at 254 nm and then classifying the 180 fractions into 14 groups; and
    (c) utilizing the method of claim 1 to determine the expression level of the reporter gene in the 14 groups so as to obtain the 6th group for activating LXRs.

17. An agonist for activating liver X receptor alpha (LXR-$\alpha$) comprising at least the 6th group as claimed in claim 16 in an effective amount and a pharmaceutically acceptable carrier.

FIG. 1

FIG. 2

FIG. 3

FIG. 4

FIG. 5

# INTERNATIONAL SEARCH REPORT

International application No.

PCT/CN2009/000587

### A. CLASSIFICATION OF SUBJECT MATTER

See extra sheet

According to International Patent Classification (IPC) or to both national classification and IPC

### B. FIELDS SEARCHED

Minimum documentation searched (classification system followed by classification symbols)

IPC:   C12Q1, C12N15, G01N33, B01D11, A61K36

Documentation searched other than minimum documentation to the extent that such documents are included in the fields searched

Electronic data base consulted during the international search (name of data base and, where practicable, search terms used)

CNPAT, WPI, EPODOC, PAJ: liver X receptor, GAL, plasmid, reporter, cimicifug+, agonist, LBD, DBD

### C. DOCUMENTS CONSIDERED TO BE RELEVANT

| Category* | Citation of document, with indication, where appropriate, of the relevant passages | Relevant to claim No. |
|---|---|---|
| X | ZHENG, Zhihui et al., Establishment of a high-throughput drug screening model for new PPAR α agonists based on mammalian one-hybrid system and study of new PPAR α agonists, Symposia of the Sixth Academic Annual Meeting of Chinese Pharmaceutical Association, 2006, see introduction and the section of material and method | 1-6 |
| A | | 7-17 |
| X | CN1980919A(IRM LLC) 13 Jun. 20071(13.06.2007), see the description, pp. 56-57 | 1-6 |
| A | | 7-17 |
| A | WO02090375A2(ARCH DEV CORP ET AL. )14 Nov. 2002(14.11.2002), see the whole document | 7-17 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 Aug. 2009 (20.08.2009) | **17 Sep. 2009 (17.09.2009)** |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>**WANG, Lihua**<br>Telephone No. (86-10)62085676 |

Form PCT/ISA/210 (second sheet) (April 2007)

# INTERNATIONAL SEARCH REPORT

| International application No. |
| --- |
| PCT/CN2009/000587 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
     because they relate to subject matter not required to be searched by this Authority, namely:

2. ☐ Claims Nos.:
     because they relate to parts of the international application that do not comply with the prescribed requirements to such an extent that no meaningful international search can be carried out, specifically:

3. ☐ Claims Nos.:
     because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

    Claims 1-6 relate to a screening method for liver X receptor agonists; claims 7-17 relate to cimicifuga extracts which could be used to activate liver X receptor. The two goups of claims are not linked by common or corresponding specific technical features, don't belong to a general technical concept and don't meet the requirement of Rule 13(1) PCT.

1. ☐ As all required additional search fees were timely paid by the applicant, this international search report covers all searchable claims.

2. ☒ As all searchable claims could be searched without effort justifying an additional fees, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**        ☐  The additional search fees were accompanied by the applicant's protest and, where applicable, the payment of a protest fee.

                           ☐  The additional search fees were accompanied by the applicant's protest but the applicable protest fee was not paid within the time limit specified in the invitation.

                           ☐  No protest accompanied the payment of additional search fees.

Form PCT/ISA/210 (continuation of first sheet (2)) (April 2007)

## INTERNATIONAL SEARCH REPORT
Information on patent family members

| International application No. |
| --- |
| PCT/CN2009/000587 |

| Patent Documents referred in the Report | Publication Date | Patent Family | Publication Date |
| --- | --- | --- | --- |
| CN1980919A | 2007-06-13 | CA2563818 A | 2005-12-08 |
| | | CA2563819A | 2005-12-08 |
| | | AU2005247931A | 2005-12-08 |
| | | AU2005247930A | 2005-12-08 |
| | | WO2005116000A | 2005-12-08 |
| | | WO2005116016A | 2005-12-08 |
| | | AR049186A | 2006-07-05 |
| | | AR049284A | 2006-07-12 |
| | | ECSP067021A | 2006-12-29 |
| | | ECSP067019A | 2006-12-29 |
| | | NO20065984A | 2007-02-05 |
| | | EP1749003A | 2007-02-07 |
| | | MXPA06013591A | 2007-03-15 |
| | | MXPA06013589A | 2007-03-15 |
| | | CN1980906A | 2007-06-13 |
| | | US2007203155A | 2007-08-30 |
| | | US2007244130A | 2007-10-18 |
| | | BRPI0511477A | 2007-12-26 |
| | | BRPI0511527A | 2008-01-02 |
| | | JP2008500354T | 2008-01-10 |
| | | JP2008500355T | 2008-01-10 |
| | | ZA200608731A | 2008-05-28 |
| | | RU2006145893A | 2008-06-27 |
| | | RU2006145894A | 2008-06-27 |
| | | ZA200608611A | 2008-08-27 |
| | | AU2009202673A | 2009-07-23 |
| WO02090375A2 | 2002-11-14 | CA2446314 A | 2002-11-14 |
| | | US2002193357A | 2002-12-19 |
| | | US7012069B | 2006-03-14 |
| | | US2003139385A | 2003-07-24 |

Form PCT/ISA/210 (patent family annex) (April 2007)

## INTERNATIONAL SEARCH REPORT
### Information on patent family members

International application No.

PCT/CN2009/000587

| Patent Documents referred in the Report | Publication Date | Patent Family | Publication Date |
|---|---|---|---|
| | | US7078396B | 2006-07-18 |
| | | US2003153541A | 2003-08-14 |
| | | EP1392713AB | 2004-03-03 |
| | | CN1585777A | 2005-02-23 |
| | | CN100360550C | 2008-01-09 |
| | | JP2005507855T | 2005-03-24 |
| | | US2007197484A | 2007-08-23 |
| | | AT375994T | 2007-11-15 |
| | | DK1392713T | 2008-02-18 |
| | | ES2296928T | 2008-05-01 |
| | | DE60223020T | 2008-07-24 |

Form PCT/ISA/210 (patent family annex) (April 2007)

## INTERNATIONAL SEARCH REPORT

International application No.

*PCT/CN2009/000587*

According to International Patent Classification (IPC) or to both national classification and IPC:

C12Q1/68    (2006.01) i
C12N15/09 (2006.01) i
G01N33/50 (2006.01) i
B01D11/00 (2006.01) i

A61K36/29    (2006.01) i

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Willy, P. J. ; Umesono, K. ; Ong, E. S. ; Evans, R. M. ; Heyman, R. A. ; Mangelsdorf, D. J.** LXR, a nuclear receptor that defines a distinct retinoid response pathway. *Genes Dev.,* 1995, vol. 9 (9), 1033-45
- **Steffensen, K.R. ; Gustafsson, J.A.** Putative metabolic effects of the liver X receptor (LXR). *Diabetes,* 2004, vol. 53 (1), 36-42
- **Proctor, G. ; Jiang, T. ; Iwahashi, M. ; Wang, Z. ; Li, J. ; Levi, M.** Regulation of renal fatty acid and cholesterol metabolism, inflammation, and fibrosis in Akita and OVE26 mice with type 1 diabetes. *Diabetes,* 2006, vol. 55 (9), 2502-9
- **Koldamova, R. P. ; Lefterov, I. M. ; Staufenbiel, M. ; Wolfe, D. ; Huang, S. ; Glorioso, J. C. ; Walter, M. ; Roth, M. G. ; Lazo, J. S.** The liver X receptor ligand T0901317 decreases amyloid beta production in vitro and in a mouse model of Alzheimer's disease. *J Biol Chem.,* 2005, vol. 280 (6), 4079-88
- **Fukuchi, J. ; Kokontis, J. M. ; Hiipakka, R.A. ; Chuu, C.P. ; Liao, S.** Antiproliferative effect of liver X receptor agonists on LNCaP human prostate cancer cells. *Cancer Res.,* 01 November 2004, vol. 64 (21), 7686-9